# EUROPEAN PATENT APPLICATION

(11) **EP 4 159 164 A1**
(43) Date of publication of application: **05.04.2023**
(21) Application number: 20937215.0
(22) Date of filing: 29.05.2020
(51) Int. Cl.: A61F 2/30

(54) **STEM FOR ARTIFICIAL JOINT**

(71) Applicant: Kyocera Corporation, Kyoto-shi Kyoto 612-8501 (JP)
(72) Inventor: NODA, Iwao, Kyoto-shi, Kyoto 612-8501 (JP)
(74) Representative: TBK
(86) International application number: PCT/JP2020/021389
(87) International publication number: WO 2021/240797

(57) **Abstract**

In the present disclosure, an artificial joint stem includes a base including one or more grooves disposed on a surface of the base, and a coating film containing a calcium phosphate-based material and an antimicrobial material disposed on a part of the surface of the base. When among the one or more grooves, the groove located in a region where the coating film is disposed is defined as a first groove, and the groove located in a region where the surface of the base is exposed is defined as a second groove, a total length of the first groove is smaller than a total length of the second groove.

## Description

### TECHNICAL FIELD

The present disclosure relates to an artificial joint stem.

### BACKGROUND OF INVENTION

The use of a biological implant for the treatment of both bone injuries and diseases is constantly expanding with the growth of the active population and aging population. In such a situation, a known biological implant is provided with a coating from the viewpoint of antimicrobial properties, adherence to bone, and the like.

For example, Patent Document 1 describes a coating for a medical implant, in which a part of the coating contains a bone-binding agent and an antimicrobial metal agent containing silver.

### CITATION LIST

### PATENT LITERATURE

Patent Document 1: JP 2011-512959 T

### SUMMARY

In the present disclosure, an artificial joint stem includes a base including one or more grooves disposed on a surface of the base, and a coating film containing a calcium phosphate-based material and an antimicrobial material disposed on a part of the surface of the base. When among the one or more grooves, the groove located in a region where the coating film is disposed is defined as a first groove, and the groove located in a region where the surface of the base is exposed is defined as a second groove, a total length of the first groove is smaller than a total length of the second groove.

Alternatively, in the present disclosure, an artificial joint stem includes a base including one or more recesses disposed on a surface of the base, a coating film containing a calcium phosphate-based material and an antimicrobial material disposed on a part of the surface of the base. When the recess located in a region where the coating film is disposed is defined as a first recess, and the groove located in a region where the surface of the base is exposed is defined as a second recess, a total opening area of the first recess is smaller than a total opening area of the second recess.

### BRIEF DESCRIPTION OF THE DRAWINGS

FIG. 1 is a schematic view illustrating an artificial joint stem according to an embodiment.
FIG. 2 is a schematic view illustrating an artificial joint stem according to an embodiment.
FIG. 3 is a schematic view illustrating a cross section of an artificial joint stem according to an embodiment.
FIG. 4 is a schematic view illustrating a cross section of an artificial joint stem according to an embodiment.
FIG. 5 is a schematic view illustrating an artificial joint stem according to an embodiment.
FIG. 6 is a schematic view illustrating an artificial joint stem according to an embodiment.
FIG. 7 is a schematic view illustrating an artificial joint stem according to an embodiment.
FIG. 8 is a schematic view illustrating a cross section of an artificial joint stem according to an embodiment.
FIG. 9 is a schematic view illustrating an artificial joint stem according to an embodiment.
FIG. 10 is a flowchart illustrating a method of manufacturing an artificial joint stem according to an embodiment.
FIG. 11 is a schematic view illustrating an artificial hip joint according to an embodiment.
FIG. 12 is a schematic view illustrating an artificial joint stem according to an embodiment.
FIG. 13 is a schematic view illustrating an artificial joint stem according to an embodiment.
FIG. 14 is a schematic view illustrating a cross section of an artificial joint stem according to an embodiment.
FIG. 15 is a schematic view illustrating an artificial joint stem according to an embodiment.
FIG. 16 is a schematic view illustrating an artificial joint stem according to an embodiment.
FIG. 17 is a schematic view illustrating an artificial joint stem according to an embodiment.

### DESCRIPTION OF EMBODIMENTS

Hereinafter, an embodiment will be described in detail. Note that, unless otherwise specified in the present specification, "A to B", which represents a numerical range, means "A or more and B or less".

### 1. Artificial Joint Stem

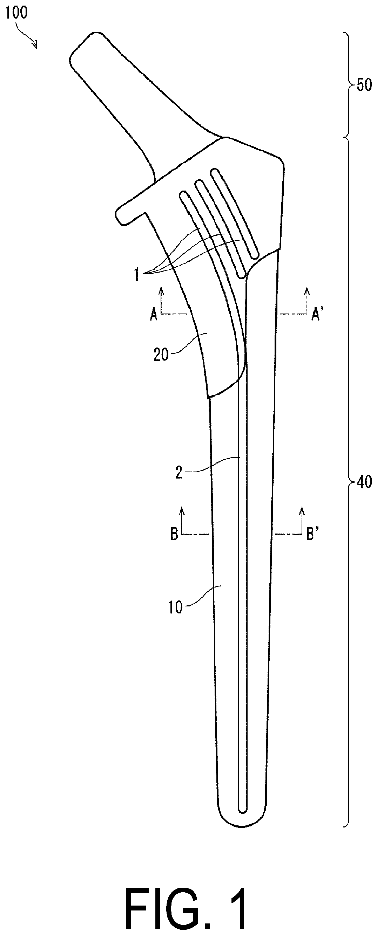

First, in an embodiment, the configuration of an artificial joint stem 100 will be described with reference to FIG. 1. The artificial joint stem 100 includes a base 10 and a coating film 20 disposed on a part of the surface of the base 10. The coating film 20 includes a calcium phosphate-based material and an antimicrobial material. The artificial joint stem 100 includes an embedded portion 40 embedded in the bone and an exposed portion 50 exposed from the bone. In FIG. 1, of the embedded portion 40, the coating film 20 is formed in a region close to the exposed portion 50, and a region far from the exposed portion 50 is exposed from the coating film 20. Note that the calcium phosphate-based material has an effect of improving adherence to bone. The antimicrobial material is also effective in reducing bacterial adhesion and growth.

The coating film 20 is disposed on a part of the surface of the base 10. That is, the other part of the surface of the base 10 is exposed from the coating film 20. For example, it is difficult to control the adherence of the artificial joint stem 100 to bone when all of the surface of the artificial joint stem 100 is covered with a coating containing a bone-binding agent and an antimicrobial metal agent. In this case, removal of the artificial joint stem may become difficult when removal of the artificial joint stem is required after surgery. For example, the embedded portion 40 may become excessively adhered to the bone through the coating. When the base 10 includes a region covered by the coating film 20 and a region exposed from the coating film 20, excessive adhesion to the bone can be reduced. That is, the adherence to bone and the antimicrobial properties can be compatible.

The base 10 includes one or more grooves disposed on the surface of the base. These grooves contribute to facilitating the insertion of the artificial joint stem 100 into the bone. Here, when among the one or more grooves, the groove located in a region where the coating film 20 is disposed is defined as a first groove 1, and the groove located in a region where the surface of the base 10 is exposed from the coating film 20 is defined as a second groove 2, the total length of the first groove 1 is smaller than the total length of the second groove 2.

In the present specification, "total length of the first groove 1" does not necessarily mean that there exists a plurality of first grooves 1. The one or more grooves may include one first groove 1 or a plurality of first grooves 1. Note that when the first groove 1 is a curved groove, the length along the curve is measured. When the first groove 1 is branched, the length of the branched grooves is also totaled. The above is true for "total length of the second groove 2". "The total length of the first groove 1 is smaller than the total length of the second groove 2" also includes a case where the total length of the first groove 1 is zero. That is, the one or more grooves mentioned above may or may not include the first groove 1. The one or more grooves may include at least the second groove 2 and may include only the second groove 2. As a result, the adherence of the artificial joint stem 100 to the bone and the like can be adjusted.

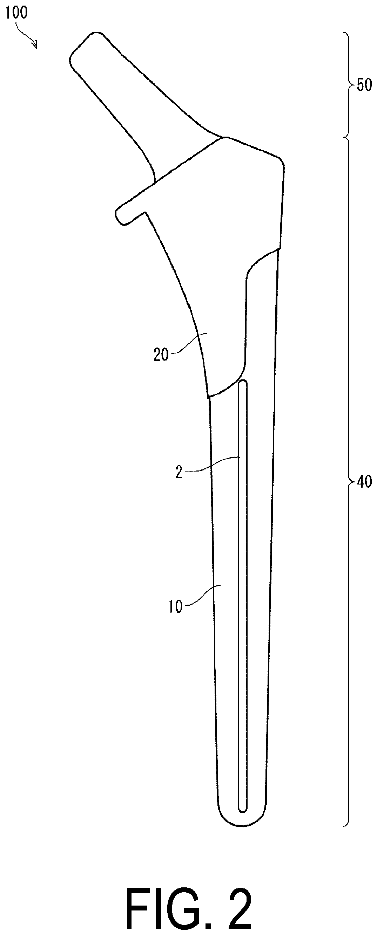

In the artificial joint stem 100 illustrated in FIG. 1, the base 10 includes both the first groove 1 and the second groove 2. In FIG. 1, the base 10 includes a plurality of first grooves 1. In FIG. 1, the base 10 includes one second groove 2. As a result, the artificial joint stem 100 can be easily inserted into the bone. FIG. 2 illustrates another example from FIG. 1. In the artificial joint stem 100 illustrated in FIG. 2, the base 10 does not include the first groove 1, but only includes the second groove 2. When the base 10 does not include the first groove 1, invasion of bacteria from the side of the region where the coating film 20 is disposed can be further reduced.

In other words, in the artificial joint stem 100, the one or more grooves include the first groove 1 located in a region where the coating film 20 is disposed, and the second groove 2 located in a region where the surface of the base 10 is exposed from the coating film 20. The total length of the first groove 1 may be smaller than the total length of the second groove 2. Alternatively, the one or more grooves may include only the second groove 2 located in the region where the surface of the base 10 is exposed from the coating film 20, and no groove need be formed in the region where the coating film 20 is disposed.

The first groove 1 may or may not be connected to the second groove 2. That is, the first groove 1 and the second groove 2 may be formed as a series of grooves. In other words, the one or more grooves may include a groove that is disposed to straddle the region where the coating film 20 is disposed and the region exposed from the coating film 20. The groove may extend to the upper end portion of the coating film 20. As illustrated in FIG. 1, only a portion of a plurality of first grooves 1 may be connected to the second groove 2. The same is true for a case where a plurality of second grooves 2 exists. When the first groove 1 is connected to the second groove 2, it is easier to insert the artificial joint stem 100 into the bone. On the other hand, when the first groove 1 is not connected to the second groove 2, invasion of bacteria from the first groove 1 side can be further reduced.

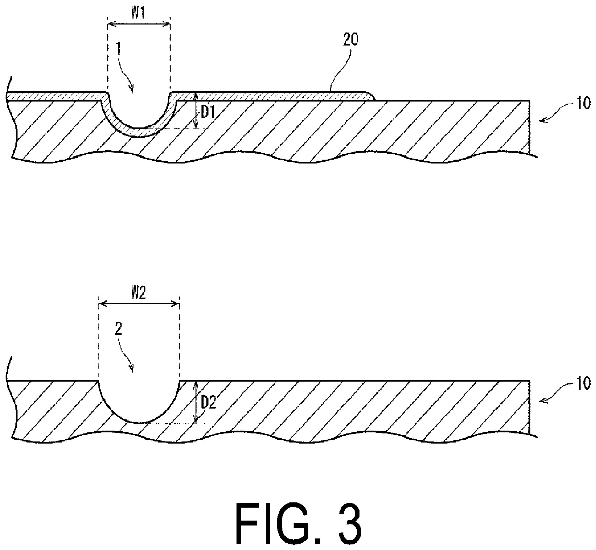

A depth D1 of the first groove 1 and a depth D2 of the second groove 2 may be the same, or either of them may be larger than the other. The same is true for a relationship between a width W1 of the first groove 1 and a width W2 of the second groove 2. Here, the depth D1 of the first groove 1 can be calculated as an average value of the depths at any number of locations in the first groove 1. The width W1 of the first groove 1 can be calculated in the same manner. The same is true for a depth D2 and a width W2 of the second groove 2. Note that the depth D1 and the width W2 of the first groove 1 represent the depth and the width on the coating film 20. FIG. 3 illustrates an A-A' cross section including the first groove 1 and a B-B' cross section including the second groove 2, in FIG. 1. In FIG. 3, the depth D1 of the first groove 1 is smaller than the depth D2 of the second groove 2. In FIG. 3, the width W1 of the first groove 1 is smaller than the width W2 of the second groove 2. When the depth D1 of the first groove 1 is smaller than the depth D2 of the second groove 2 and/or the width W1 of the first groove 1 is smaller than the width W2 of the second groove 2, invasion of bacteria from the first groove 1 side can be further reduced.

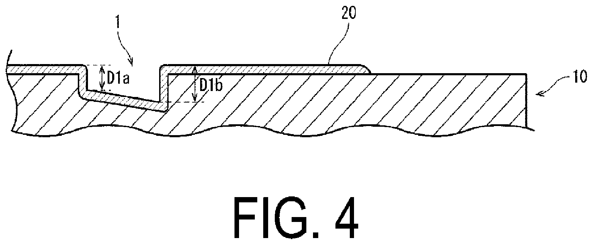

At least one of the one or more grooves may each have the same depth at both end portions in the width direction of the respective groove, or the depth at one end portion may be smaller than that at the other end portion. FIG. 4 illustrates an A-A' cross section including the first groove 1 in FIG. 1, which is a different example from FIG. 3. In FIG. 4, a depth D1a at one end portion of the first groove 1 is smaller than a depth D1b at the other end portion in the width direction of the first groove 1.

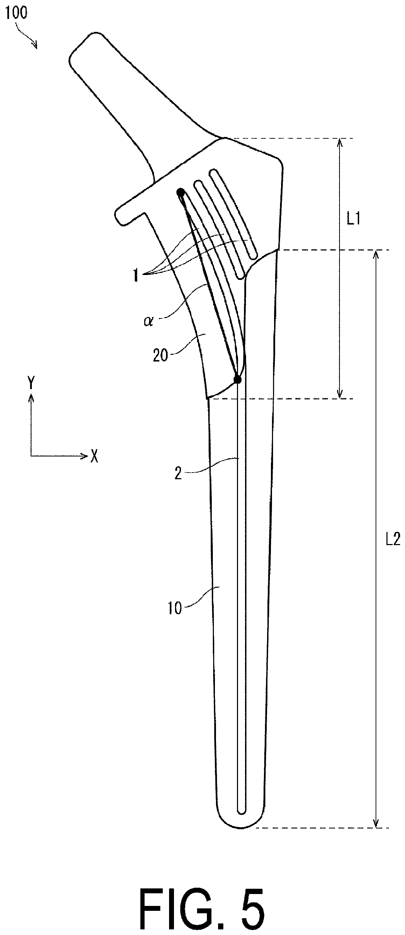

The one or more grooves can each also be broken down into a component along the width direction of the base 10 and a component along the direction orthogonal to the width direction. The components of each of the one or more grooves will be described below with reference to FIG. 5. The base 10 illustrated in FIG. 5 has a shape extending in the vertical direction. For the vertical direction of the base 10, the upward direction corresponds to the proximal direction and the downward direction corresponds to the distal direction of a human body. The width direction of the base 10 can be a direction orthogonal to the vertical direction. That is, the direction orthogonal to the width direction may be the vertical direction of the base 10. In FIG. 5, the width direction of the base 10 is represented as the X-axis direction, and the direction orthogonal to the width direction is represented as the Y-axis direction.

Here, among the straight lines that connect the end portions of respective first grooves 1, the straight line α has a maximum length, and will be examined. When the line α is parallel to the X axis, the first groove 1 of the line α has only a component along the width direction of the base 10. When the line α is parallel to the Y axis, the first groove 1 has only a component along the direction orthogonal to the width direction of the base 10. When the straight line α is represented as a straight line having an inclination on the XY coordinates, the first groove 1 has both a component along the width direction of the base 10 and a component along the direction orthogonal to the width direction.

The first grooves 1 illustrated in FIG. 5 each have both a component along the width direction of the base 10 and a component along the direction orthogonal to the width direction, but may have either one. The first groove 1 has at least one of a component along the width direction of the base 10 and a component along the direction orthogonal to the width direction. The first groove 1 having a component along the width direction of the base 10 means that the first groove 1 is not parallel to the vertical direction. In other words, the first groove 1 is not parallel to the insertion direction of the artificial joint stem 100 into the bone, but is inclined. When the first groove 1 has a component along the width direction of the base 10, it is possible to resist sinking, which is a phenomenon in which the artificial joint stem sinks downward after being inserted. The second groove 2 may also have both a component along the width direction of the base 10 and a component along the direction orthogonal to the width direction.

The component along the width direction of the base 10 may have the same size as the component along the direction orthogonal to the width direction, or one of them may be larger than the other. For example, as illustrated in FIG. 5, the first groove 1 may have a component along the width direction of the base 10 smaller than a component along the direction orthogonal to the width direction. Here, "the component along the width direction of the base 10 is smaller than the component along the direction orthogonal to the width direction" means that the absolute value of the inclination of the straight line α is greater than 1. In this case, the artificial joint stem 100 can be more easily inserted into the bone. Similarly to the second groove 2, the component along the width direction of the base 10 may be smaller than the component along the direction orthogonal to the width direction.

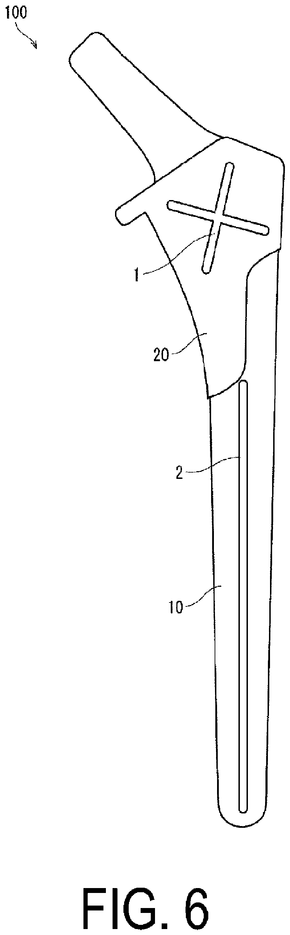

At least one of the one or more grooves may each have a linear portion or a curved portion. For example, the first groove 1 may include a plurality of linear portions. The plurality of linear portions may be parallel to each other, intersect each other at a right angle, or be randomly disposed. FIG. 6 illustrates the artificial joint stem 100 in which the first groove 1 includes a plurality of linear portions that intersect each other at a right angle.

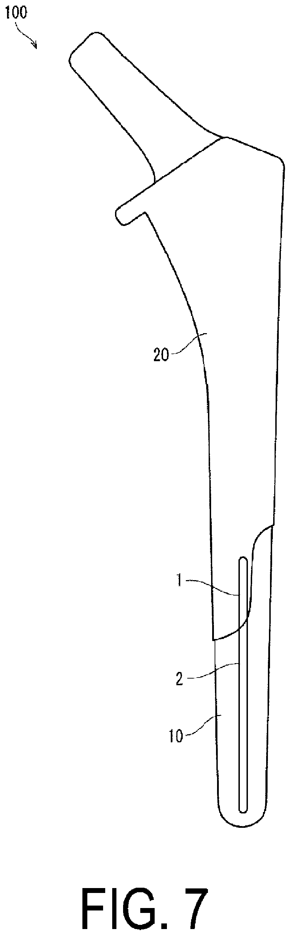

Of the surface of the base 10, the ratio between the area of a region where the coating film 20 is disposed and the area of a region where the surface of the base 10 is exposed from the coating film 20 may be appropriately determined depending on the application, or the like. The area of the region where the coating film 20 is disposed may be the same as the area of the region where the surface of the base 10 is exposed from the coating film 20, or one of the regions may be larger than the other. For example, in FIG. 1, the area of the region where the coating film 20 is disposed is smaller than the region where the surface of the base 10 is exposed from the coating film 20. In this case, the artificial joint stem 100 can be more easily inserted into the bone. On the other hand, FIG. 7 illustrates the artificial joint stem 100 where the area of the region where the coating film 20 is disposed is greater than the area of the region where the surface of the base 10 is exposed from the coating film 20. In this case, the antimicrobial properties can be improved.

Within the surface of the base 10, L1 is defined as a length along the orthogonal direction orthogonal to the width direction of the base 10 in the region where the coating film 20 is disposed. L2 is defined as a length along the orthogonal direction in the region where the surface of the base 10 is exposed from the coating film 20. The ratio of L1 and L2 can also be appropriately determined according to the application or the like. The length L1 may be the same as the length L2, or one of them may be larger than the other. For example, in FIG. 5, the length L1 is smaller than the length L2. In this case, the artificial joint stem 100 can be more easily inserted into the bone. Here, the length L1 represents the maximum value of the length along the Y axis direction in the region where the coating film 20 is disposed. That is, the length L1 represents a difference in the Y coordinate between a point where the Y coordinate is maximum and a point where the Y coordinate is minimum, in the region where the coating film 20 is disposed. The length L2 also represents the maximum value of the length along the Y axis direction in the region where the surface of the base 10 is exposed from the coating film 20. That is, the length L2 represents a difference in the Y coordinate between a point where the Y coordinate is maximum and a point where the Y coordinate is minimum, in the region where the surface of the base 10 is exposed from the coating film 20.

As the base 10, a metal, ceramic or plastic may be used. Examples of the metal include stainless steel alloy, cobalt-chromium alloy, titanium, and titanium alloy. The titanium alloy can be made by adding at least one selected from the group consisting of aluminum, tin, zirconium, molybdenum, nickel, palladium, tantalum, niobium, vanadium, and platinum to titanium. Examples of the ceramic include alumina, zirconia and alumina-zirconia composite ceramic. Examples of the plastic include polyethylene, fluorine-based resin, epoxy resin, polyetheretherketone (PEEK) resin, and bakelite. Note that, in the present embodiment, the base 10 is made of a titanium alloy.

The shape of the base 10 may be substantially rod-shaped, for example, but may be appropriately changed according to the shape of the artificial joint to be applied.

The coating film 20 includes a calcium phosphate-based material and an antimicrobial material. Examples of the calcium phosphate-based material include one or more types of mixtures selected from the group consisting of hydroxyapatite, α-tertiary calcium phosphate, β-tertiary calcium phosphate, quaternary calcium phosphate, octacalcium phosphate, and calcium phosphate-based glass. As the antimicrobial material, a natural antimicrobial agent, an organic antimicrobial agent, or an inorganic antimicrobial agent can be used. For example, hinokitiol can be used as a natural antimicrobial agent, benzalkonium chloride can be used as an organic antimicrobial agent, and a metal can be used as an inorganic antimicrobial agent. Examples of the metal include silver, copper, and zinc. In addition to the calcium phosphate-based material and the antimicrobial material, the coating film 20 may contain a glass ceramic, and may further contain an antimicrobial agent such as penicillin and vancomycin.

The concentration of the antimicrobial material in the coating film 20 may be, for example, from 0.05 wt% to 3.00 wt%, from 0.05 wt% to 2.50 wt%, from 0.05 wt% to 1.00 wt%, or from 0.1 wt% to 1.00 wt%. When the concentration of the antimicrobial material is 0.05 wt% or more, sufficient antimicrobial properties can be achieved. When the concentration of the antimicrobial material is 3.00 wt% or less, the impact on living tissue can be reduced.

There may be a concentration gradient of antimicrobial material in the coating film 20. For example, the concentration of the antimicrobial material contained in an upper end portion of the coating film 20 may be greater than the concentration of the antimicrobial material contained in a lower end portion of the coating film. Thus, invasion of bacteria from the upper end portion side of the coating film 20 can be more effectively reduced. The antimicrobial material may be contained only at the upper end portion of the coating film 20.

On the base 10, there may exist one or more boundary lines defined by the presence or absence of the coating film 20. At least one of the one or more boundary lines each has a circumferential length around the base 10. The circumferential length may be larger than a circumferential length of a part of the base 10 located above and below the respective boundary line. For example, a part where the at least one boundary line exists may be raised in a node shape on the base 10.

The regions where the coating film 20 is disposed and the regions exposed from the coating film 20 can be distinguished by elemental analysis of the surface of each region. The method of elemental analysis can be performed, for example, by mapping the surface elements with an energy dispersive X-ray spectrometry (EDX) apparatus, which is an accessory for a general scanning electron microscope (SEM). Surface analysis methods such as X-ray photoelectron spectroscopy, Auger electron spectroscopy, and secondary ion mass spectrometry may also be used. The element may be confirmed by chemical analysis of a sample obtained by mechanically scraping off the surface of each region. For example, in the region where the coating film 20 is disposed, phosphorus, calcium, antimicrobial components, or the like are detected. On the surface of the region exposed from the coating film 20, elements constituting the base 10 are detected, and phosphorus, calcium, antimicrobial components or the like are not detected, or the detected level is below the noise level.

The base 10 may have a rough surface located on the surface. The rough surface may be covered with the coating film 20 or exposed from the coating film 20. Only a part of the rough surface may be covered with the coating film 20, and the other part of the rough surface may be exposed from the coating film 20. The one or more grooves may or may not be located on the rough surface. For example, only the first groove 1 may be located on the rough surface. The surface roughness of the inner surface of the groove located on the rough surface may be smaller than the surface roughness of the rough surface of the base 10.

An example of an index of the surface roughness of the rough surface includes an arithmetic mean roughness Sa (ISO 25178). The surface roughness (Sa) of the rough surface may be set to, for example, from 10 to 80 µm, from 20 to 80 µm, or from 30 to 70 µm. Note that the surface roughness (Sa) of the rough surface can be measured, for example, by cutting the artificial joint stem 100 and observing the cut surface by SEM or the like.

The surface roughness (Sa) is only required to be measured, for example, by a stylus method or an optical method. The surface roughness (Sa) is only required to be measured in accordance with "ISO 25178", for example. Note that the measurement of the surface roughness (Sa) is not limited to the above-described method.

The artificial joint stem 100 may further include the layer member 30. As used herein, "layer member" means a member different from the coating film 20 that is layered on the base 10. For example, the surface of the layer member 30 may be a rough surface. Thus, the region primarily in contact with the bone can have a rough surface. The layer member 30 may be formed by a thermal spraying method as described below. Alternatively, the layer member 30 may be formed as a porous structure.

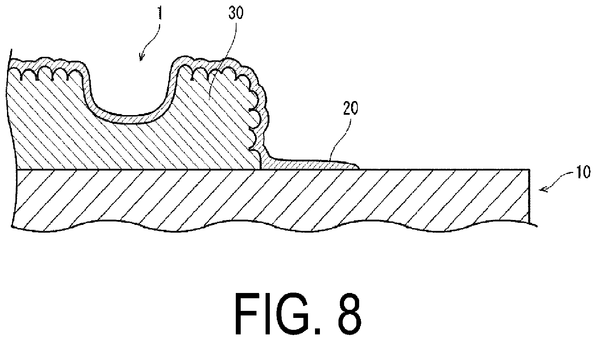

FIG. 8 illustrates an A-A' cross section including the first groove 1, which is a different example from FIG. 3 and 4. In FIG. 8, a layer member 30 is provided as a rough surface. Thus, the region where the layer member 30 is provided is higher than the region where the layer member 30 is not provided. Accordingly, when the artificial joint stem 100 is embedded in the bone, the layer member 30 can mainly be brought into contact with the bone. Note that in FIG. 8, a coating film 20 is formed on the layer member 30, and only the first groove 1 is located as the groove.

Note that the lower limit of the height of the layer member 30 may be only required to be set to, for example, 100 µm or more, and may be set to 300 µm or more. The upper limit may be only required to be set to, for example, 1000 µm or less, and may be set to 700 µm or less. The surface roughness of the layer member 30 may be set to, for example, 10 to 80 µm, 20 to 80 µm, or 30 to 70 µm.

As the material of the layer member 30, the material exemplified as the material of the base 10 can be used. For example, the layer member 30 may be made of a metal. Thus, sufficient strength can be secured. The material of the layer member 30 and the material of the base member 10 may be the same or different. Note that in the present embodiment, the layer member 30 is made of a titanium alloy.

The height of the layer member 30 may be greater than the thickness of the coating film 20. Thus, since the region where the layer member 30 is formed is higher than the region where only the coating film 20 is formed, the region where the layer member 30 is formed can mainly be brought into contact with the bone. The thickness of the coating film 20 may be only required to be set to, for example, less than 100 µm, and may be set to less than 50 µm. The coating film 20 may be only required to be, for example, set to be 5 µm or more.

Although the artificial joint stem 100 having the one or more grooves has been described above, the shape of the groove is not particularly limited. For example, instead of a groove, a recess having a circular, polygonal, or indeterminate form may be formed. The artificial joint stem 100 in which such a recess is formed is also included in the present disclosure. In the present disclosure, an artificial joint stem 100 includes a base 10 including one or more recesses disposed on a surface of the base 10, and a coating film 20 including a calcium phosphate-based material and an antimicrobial material disposed on a part of the surface of the base 10. When the recess located in a region where the coating film 20 is disposed is defined as a first recess, and the recess located in a region where the surface of the base 10 is exposed from the coating film 20 is defined as a second recess, the total opening area of the first recess may be smaller than the total opening area of the second recess. The relationship between the first recess and the second recess can be similar to the relationship between the first groove 1 and the second groove 2 described above. The opening areas of the first and second recesses can be calculated by using image analysis software or the like.

Also, as described above, the base 10 may include the embedded portion 40 embedded in the bone and the exposed portion 50 exposed from the bone. The femur is an example of the bone. A coating film 20 may be formed on a part of the peripheral wall of the embedded portion 40. Thus, the embedded portion 40, which can actually come into contact with the bone, can exhibit desirable adherence and antimicrobial properties.

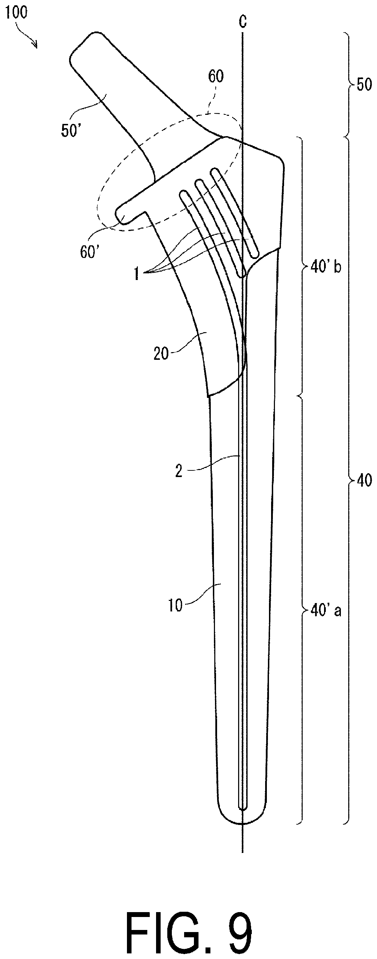

For example, as illustrated in FIG. 9, at least a part of the coating film 20 may be disposed in a boundary region 60 including a boundary between the embedded portion 40 and the exposed portion 50. That is, the coating film 20 may be disposed in a region of the embedded portion 40 near the exposed portion 50. Thus, invasion of bacteria from the exposed portion 50 side can be more effectively reduced.

The coating film 20 disposed in the boundary region 60 may be disposed only in the embedded portion 40. That is, the coating film 20 need not be disposed in the exposed portion 50. Thus, irritation to the soft tissue that may come in contact with the exposed portion 50 can be reduced.

Note that the first groove 1 may not reach the boundary between the embedded portion 40 and the exposed portion 50. In other words, the ends of the first groove 1 may be located at the embedded portion 40. In this case, the invasion of bacteria can be further reduced.

As illustrated in FIG. 9, the base 10 may include a body portion 40' and a neck portion 50' connected to an upper end portion of the body portion 40'. The body portion 40' may be embedded in the femur. The neck portion 50' may be exposed from the femur. The neck portion 50' may be provided with a bone head. The bone head may be fitted into an acetabular cup that constitutes a pair with the artificial joint stem.

The body portion 40' includes a lower portion 40'a and an upper portion 40'b. The lower portion 40'a has a center axis C extending along a vertical direction. The upper portion 40'b extends in the vertical direction continuously from the lower portion 40'a and bends such that the center of the upper portion 40'b gradually departs further away from the center axis C as the upper portion 40'b extends upward. The upper portion 40'b includes an upper end surface disposed offset from the center axis C, and the upper end surface is connected to the neck portion 50'. The neck portion 50' is smaller in width than the upper end face of the body portion 40'. In other words, the neck portion 50' can be a protruding portion 50' protruding in an oblique direction inclined from the center axis C of the body portion 40'.

The base 10 may further include a collar 60' provided at a connecting portion between the body portion 40' and the neck portion 50'. The collar 60' is a protruding portion protruding from the connecting portion toward the surface direction of the upper end face. The collar 60' can reduce excessive penetration of the body portion 40' into the bone during surgery to insert the artificial joint stem into the bone.

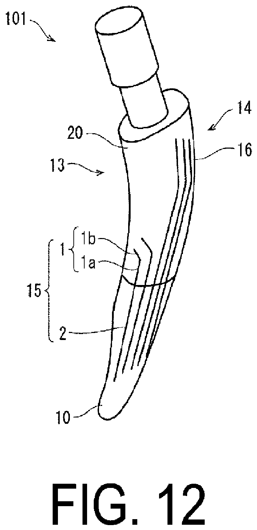

In the present disclosure, the artificial joint stem also includes an artificial joint stem 101 illustrated in FIG. 12. For example, the base 10 may include an inner side portion 13 that curves concavely and an outer side portion 14 that curves convexly. An upper end portion of the first groove 1 may be located at a bend on the inner side portion side. Here, the upper end portion of the first groove 1 may be bent toward the inner side portion 13 side. In other words, the first groove 1 may include a first portion 1a extending in the vertical direction of the base 10 and a second portion 1b connected to the first portion 1a and having a component along the width direction of the base 10. The depth of the second groove 2 may be smaller than the depth of the first groove 1.

The base 10 may also include a first groove set 15 and a second groove set 16. The first groove set 15 includes the first groove 1 and the second groove 2 connected to each other. The second groove set 16 includes the first groove 1 and the second groove 2 connected to each other, and the first groove 1 extends to the upper end portion of the coating film 20 further than the first groove set 15.

In addition, the base 10 may include a plurality of first groove sets 15. The plurality of the first groove sets 15 may be arranged in the width direction of the base 10. Of the plurality of the first groove sets 15, the first groove set 15 located on the outer side portion 14 side may be located above the first groove set 15 located on the inner side portion 13 side.

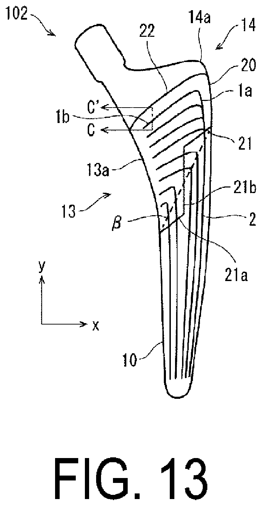

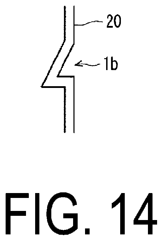

In the present disclosure, the artificial joint stem also includes an artificial joint stem 102 illustrated in FIG. 13. For example, the base 10 may include a plurality of grooves, and a groove located on the upper portion of the base 10 may be wider than a groove located on the lower portion of the base 10. The grooves may have a component along the width direction of the base 10. FIG. 14 illustrates a C-C' cross section of FIG. 13. As illustrated in FIG. 14, the groove (second portion 1b of the first groove 1) along the width direction of the base 10 may be shallower upward.

One or more boundary lines are defined by the presence or absence of the coating film 20. The one or more boundary lines may include a first boundary line 21 located on the lower side of the base 10 with respect to the coating film 20. The one or more boundary lines may also include a second boundary line 22 located on the upper side of the base 10 with respect to the coating film 20. The first boundary line 21 may intersect a linear portion of the groove. Here, the first boundary line 21 may diagonally intersect the linear portion of the groove. That is, the first boundary line 21 may not be orthogonal to the linear portion of the groove.

The boundary line may have a component along the vertical direction larger than a component along the width direction of the base 10. That is, when viewed in a plan view from a direction perpendicular to the XY plane of FIG. 13, the inclination of a straight line connecting both ends of the boundary line on the XY coordinates may exceed 1. In FIG. 13, the inclination of the straight line β connecting both ends of the first boundary line 1 on the XY coordinates exceeds 1.

The first boundary line 21 may include a first portion 21a that extends in a direction intersecting the groove and a second portion 21b that extends in a direction along the groove. The second portion 21b of the first boundary line 21 may be separated from the groove. That is, the second portion 21b of the first boundary line 21 need not be in contact with the groove. The same is true for the second boundary line 22.

The first groove 1 and the second groove 2 may be connected. The first groove 1 may include the first portion 1a extending in the vertical direction of the base 10 and the second portion 1b connected to the first portion 1a and having a component along the width direction of the base 10. As illustrated in FIG. 13, the second boundary line 22 may extend in a direction along the second portion 1b of the first groove 1.

As illustrated in FIG. 13, the second boundary line 22 may be disposed to incline upward from the inner side portion 13 toward the outer side portion 14. As illustrated in FIG. 13, the first boundary line 21 may be located downward of an apex 13a of the recessed portion at the inner side portion 13. The first boundary line 21 may be located downward of an apex 14a of the protruding portion at the outer side portion 14. Alternatively, the first boundary line 21 may be located upward of the apex 14a of the protruding portion at the outer side portion 14.

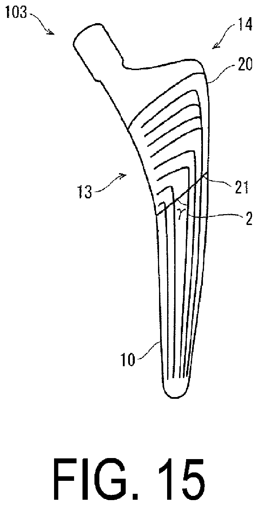

In the present disclosure, the artificial joint stem also includes an artificial joint stem 103 illustrated in FIG. 15. For example, the boundary line may intersect the groove at a sharp angle. In FIG. 15, among the angles formed by the first boundary line 21 and the second groove 2, an angle γ on the inner side portion 13 side is a sharp angle.

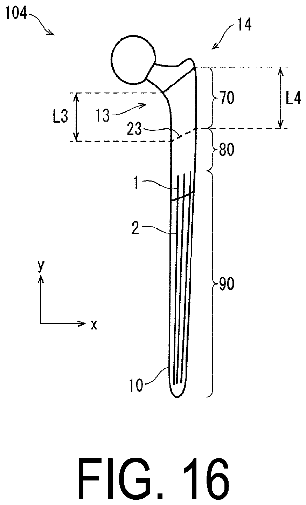

In the present disclosure, the artificial joint stem also includes an artificial joint stem 104 illustrated in FIG. 16. For example, the base 10 includes, from top to bottom, a rough-surface region 70, a non-rough-surface region 80, and a groove region 90. A rough surface is disposed in the rough-surface region 70. The non-rough-surface region 80 is a region without a rough surface. The grooves are disposed in the groove region 90. For example, the rough-surface region 70 may be a region in which rough surfaces are disposed but no grooves are disposed. The non-rough-surface region 80 may be a region in which neither a rough surface nor a groove is disposed. The groove region 90 may be a region in which a rough surface is not disposed but a groove is disposed. The coating film 20 may cover at least one selected from the group consisting of the rough-surface region 70, the non-rough-surface region 80 and the groove region 90. The area of the rough-surface region 70 may be smaller than that of the non-rough-surface region 80. In the width direction of the base 10, the length of the rough-surface region 70 may be greater than the length of the non-rough-surface region 80. One or more boundary lines 23 between the rough-surface region 70 and the non-rough-surface region 80 may be inclined upward from the inner side portion 13 toward the outer side portion 14. A length L3 on the inner side portion 13 side of the rough-surface region 70 may be less than the length L4 on the outer side portion 14 side of the rough-surface region 70. Here, when viewed in a plan view from a direction perpendicular to the XY plane of FIG. 16, the length L3 represents a difference in the Y coordinate between a point where the Y coordinate is maximum and a point where the Y coordinate is minimum, on the inner side portion 13 side of the rough-surface region 70. A length L4 represents a difference in the Y coordinate between a point where the Y coordinate is maximum and a point where the Y coordinate is minimum, on the outer side portion 14 side of the rough-surface region 70.

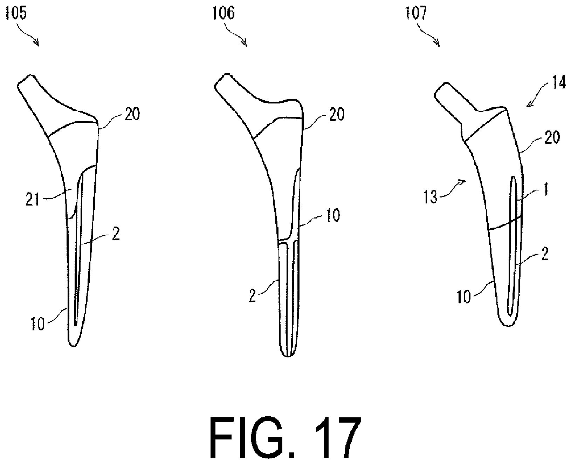

In the present disclosure, the artificial joint stem also includes artificial joint stems 105, 106, and 107 illustrated in FIG. 17. For example, as in the artificial joint stem 105, only the second boundary line 2 may be along a part of the first boundary line 21. As in the artificial joint stem 106, a plurality of second grooves 2 may be disposed in a circumferential direction at a distal end portion of the base 10. The coating film 20 and the second groove 2 need not be in contact with each other. As in the artificial joint stem 107, the groove may be closer to either the inner side portion 13 or the outer side portion 14. In FIG. 17, the first groove 1 and the second groove 2 of the artificial joint stem 107 are closer to the outer side portion 14.

### 2. Method of Manufacturing Artificial Joint Stem

In an embodiment, a method of manufacturing an artificial joint stem includes, for example, a preparation step, a groove forming step, and a coating film forming step. In the preparation step, a base 10 having a surface including a first region and a second region is prepared. The groove forming step is a step of forming one or more grooves on the surface of the base 10. The coating film forming step is a step of forming the coating film 20 containing a calcium phosphate-based material and an antimicrobial material on a part (the first region) of the surface of the base 10.

In the artificial joint stem of the present disclosure, the groove located in the region where the coating film 20 is disposed is the first groove 1, and the groove located in the region where the surface of the base 10 is exposed from the coating film 20 is the second groove 2. That is, in the groove forming step, the groove formed in the first region is the first groove 1, and the groove formed in the second region is the second groove. In the groove forming step, the one or more grooves are formed such that the total length of the first groove 1 is smaller than the total length of the second groove 2. In the coating film forming step, a coating film 20 is formed in a first region of the surface of the base 10 where the first groove 1 is formed. Thus, the artificial joint stem 100 described above can be obtained.

In the preparation step, the base 10 can be prepared by molding the metal material into a desired shape by using a metal mold, or an additive manufacturing method, or the like.

In the groove forming step, the groove can be formed by at least one selected from the group consisting of a cutting method, a rolling processing method and a pressing method. Note that, in the present embodiment, a groove is formed by milling, for example, which is one of the cutting methods. When the artificial joint stem includes the recess described above, a recess forming step may be only required to be provided in place of the groove forming step. The recess forming step uses a method similar to the groove forming step.

After the groove forming step, a coating film forming step can be performed. The coating film 20 can be formed by: a thermal spraying method such as flame spraying, highspeed flame spraying, and plasma spraying; a physical vapor deposition method or chemical vapor deposition method such as sputtering, ion plating, ion beam deposition, and an ion mixing method; or a wet coating method such as a sol-gel method. Note that the material constituting the coating film is also referred to as a coating material.

A first protective material may be used to form the coating film 20 only in the first region. In this case, the coating film forming step may further include a step of disposing the first protective material so as to protect the second region while exposing the first region so that the coating film is not formed in the second region. For example, a masking tape or a screen may be used as the first protective material. Alternatively, a jig covering the base 10 may be used as the first protective material. Examples of the first protective material include metals, glasses, resins, and composite materials thereof. Note that the first protective material may or may not be in contact with the base 10. When, for example, a masking tape is used as the first protective material, the first protective material may be disposed on the second region. When a jig covering the base 10 is used, the shape of the jig is not particularly limited, but may be, for example, tubular. The cross section of the tubular jig may be polygonal or circular.

When a screen is to be disposed, the coating film 20 can be formed in a specific region by placing the screen in a predetermined location. When a jig is used, the coating film 20 can be formed in a specific region by placing the jig in a predetermined location. In this case, for example, the coating film 20 can be selectively formed only in a desired region by adjusting the positional relationship between the screen and a discharge nozzle configured to discharge the thermal spraying material, the additive manufacturing material, the chemical etching material, the blasting material, or the coating material. In this case, a tip of the discharge nozzle is only required to be disposed, for example, in a straight line with the surface of the desired region without being separated by the screen. Hereinafter, a thermal spraying material, an additive manufacturing material, a chemical etching material, a blasting material, or a coating material discharged from the discharge nozzle is also referred to as a discharge material. Without being limited to the above, the coating film 20 may be formed while the base 10, the screen, and the discharge nozzle are fixed, or the coating film 20 may be formed while moving at least one selected from the group consisting of the aforementioned. The angle of the discharge nozzle may be fixed or the coating film 20 may be formed while changing the angle.

Note that the coating film 20 can be formed only in a desired region without using a protective material. For example, the coating film 20 can be selectively formed only in a desired region by adjusting the shape, angle degree, or position, of the discharge nozzle configured to discharge the discharge material. For example, the discharge material may be discharged with the discharge nozzle located above the surface of the desired region. In this case, the coating film 20 may be formed by fixing the base 10 and moving the position and angle of the discharge nozzle, or the coating film 20 may be formed by fixing the discharge nozzle and moving the position and angle of the base 10. The discharge nozzle may be moved at a constant speed or at a variable speed. The discharge direction of the discharge material may be an angle of 90 ° or an angle of less than 90 ° with the vector extending from the tip of the discharge nozzle toward the base 10 or the surface of the rough surface, which are located at the shortest distance from the tip of the discharge nozzle.

A roughening step may be performed before the groove forming step. The roughening step is a step of forming a rough surface on the base 10. Specifically, in the preparation step, the base 10 having a surface further including a roughening region in addition to the first region and the second region is prepared. Then, in the roughening step, a rough surface is formed in the roughening region of the base 10.

In the roughening step, a rough surface can be formed by at least one selected from the group consisting of a thermal spraying method, an additive manufacturing method, a chemical etching method, and a blasting method. Compared with the blasting method, the thermal spraying method, the additive manufacturing method or the chemical etching method can increase the surface roughness. Note that the material discharged toward the base 10 in the thermal spraying method is referred to as a thermal spraying material. Similarly, a material discharged toward the base 10 in the additive manufacturing method is referred to as an additive manufacturing material. A material discharged toward the base 10 when processing by the chemical etching method is referred to as a chemical etching material. Similarly, a material discharged toward the base 10 when processing by the blasting method is referred to as a blasting material. As the thermal spraying material and the additive manufacturing material, the material exemplified as the material for the base 10 can be used. The layer member described above may be formed by a thermal spraying method or an additive manufacturing method. Examples of the chemical etching method include alkali treatment. Examples of the blasting method include sandblasting.

A second protective material may be used to form a rough surface only in a desired region. In this case, the roughening step may further include a step of disposing the second protective material so as to protect other regions while exposing the roughening region so that no rough surface is formed in other than the roughening region. Note that the roughening region may be located inside the first region or may straddle across the first region and the second region. That is, the second protective material may be disposed to protect a part of the first region and the second region, or the second region.

For example, a masking tape or a screen may be used as the second protective material. Alternatively, a jig covering the base 10 may be used as the second protective material. Examples of the second protective material include metals, glasses, resins, and composite materials thereof. Note that the protective material may be or need not be in contact with the base 10. When a jig covering the base 10 is used as the second protective material, the shape of the jig is not particularly limited, but may be, for example, tubular. The cross section of the tubular jig may be polygonal or circular.

When a screen is to be placed, a rough surface can be formed in a specific region by placing the screen in a predetermined position. When a jig is to be used, a rough surface can be formed in a specific region by placing the jig in a predetermined position. In this case, for example, the rough surface can be selectively formed only in a desired region by adjusting the positional relationship between the screen and a discharge nozzle for discharging the thermal spraying material, the additive manufacturing material, the chemical etching material, the blasting material, or the coating material. In this case, a tip of the discharge nozzle is only required to be disposed, for example, in a straight line with the surface of the desired region without being separated by the screen. Hereinafter, a thermal spraying material, an additive manufacturing material, a chemical etching material, a blasting material, or a coating material discharged from the discharge nozzle is also referred to as a discharge material. Without being limited to the above, the rough surface may be formed while the base 10, the screen, and the discharge nozzle are fixed, or the rough surface may be formed while moving at least one selected from the group consisting of the aforementioned. The angle of the discharge nozzle may be fixed or the rough surface may be formed while changing the angle. Note that similarly to the coating film 20, a rough surface may be formed only in a desired region without using a protective material.

As described above, when the artificial joint stem of the present disclosure includes a rough surface, for example, in the roughening step, while exposing a part of the surface of the base 10, the second protective material may be disposed on the base 10 so as to protect another part of the base 10, so that a rough surface may be formed on the exposed part of the surface. In the present disclosure, the manufacturing method may further include a step of removing the second protective material after the roughening step and before the groove forming step.

After the second protective material is removed, a step of scraping the edge of the rough surface, for example, the edge of the layer member 30, may be performed. Thus, the concentration of stress at the edge of the layer member 30 can be avoided, and irritation to the biotissue can be reduced.

A second masking tape may be used as the second protective material. In this case, in the present disclosure, the manufacturing method may further include a step of, while exposing a part of the base 10, sticking the second masking tape to another part of the base 10, before the roughening step.

In the coating film forming step, while exposing a part of the surface of the base 10, the first protective material may be disposed on the base 10 so as to protect another part of the base 10, so that the coating film 20 may be formed on the exposed part of the surface. The first protective material can be removed after the coating film forming step. A first masking tape may be used as the first protective material. In this case, in the present disclosure, the manufacturing method may further include a step of, while exposing a part of the base 10, attaching a first masking tape to another part of the base 10, before the coating film forming step.

As the second protective material, one having higher thermal resistance than that of the first protective material can be used. For example, a material which is not melted or pyrolyzed for 1 minute under a thermal spraying condition of 8000°C may be used as the second protective material, and a material which is not melted or pyrolyzed for 1 minute under a thermal spraying condition of 3000°C may be used as the first protective material. A specific example of such a material includes a composite material of glass and resin.

When processing by the blasting method in the roughening step, a material that is not dissolved or pyrolyzed at room temperature may be used as the second protective material. A specific example of such a material includes a resin.

In the present disclosure, in the step of forming the rough surface or the coating film 20 in the manufacturing method, a protective material may be disposed in addition to the first protective material and the second protective material. For example, in the step of forming the rough surface or the coating film 20, a protective material may be disposed on a part or all of the exposed portion 50. Thus, the presence or absence of the formation of the rough surface or the coating film 20 in the exposed portion 50 can be appropriately controlled. For example, by disposing a protective material in a region of the exposed portion 50 far from the embedded portion 40 and forming the coating film 20 in the exposed region, the coating film 20 can be formed in a region of the exposed portion 50 near the embedded portion 40.

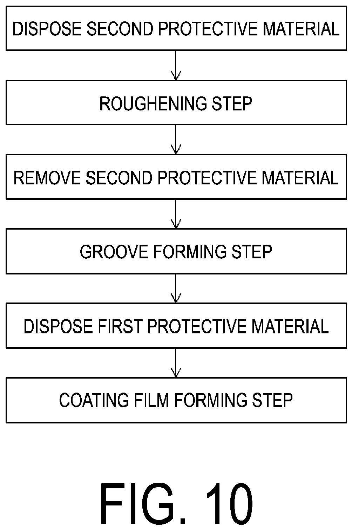

In summary, each step can be performed in the order illustrated in FIG. 10, for example. FIG. 10 is a flowchart illustrating a method of manufacturing an artificial joint stem 100 according to an embodiment. First, the second protective material is disposed, and after a roughening step is performed, the second protective material can be removed. Thereafter, the groove forming step can be performed. Then, the first protective material may be disposed and then the coating film forming step may be performed.

In the present disclosure, the manufacturing method may or may not include a cleaning step between each step. For example, in the present disclosure, the manufacturing method includes a step of cleaning the base 10, or the base 10 and the layer member 30 after the roughening step. The cleaning method is not particularly limited, but may be, for example, a method of immersing in a liquid such as water or an organic solvent such as alcohol, or a method of showering using the liquid. Alternatively, a method of blowing a gas such as air, nitrogen or argon may be employed. Thus, the excess thermal spraying material or the like and/or scrapes or the like generated by the roughening step can be removed.

Although the manufacturing method of the artificial joint stem 100 including the groove has been described above, the manufacturing method according to the present disclosure is not particularly limited to the steps described above. For example, in the manufacturing method described above, an example has been described where after the roughening step of roughening the surface of the base 10, a groove forming step of forming a groove is performed, and then the coating film forming step of forming a coating film is performed. Instead, after the groove forming step, the roughening step may be performed, and then the coating film forming step may be performed.

The roughening step may include, in order, a first roughening step of forming a first rough surface by a thermal spraying method and a second roughening step of forming a second rough surface by a chemical etching method or a blasting method. Herein, a region where the first rough surface is formed by thermal spraying method is referred to as a first roughening region, a region where the second rough surface is formed by chemical etching method or blasting method is referred to as a second roughening region, and a region where no rough surface is formed is referred to as a non-roughening region.

In the first roughening step, a third protective material (the second protective material described above) may be disposed to protect the second roughening region and the non-roughening region with respect to the base 10 while exposing the first roughening region, thereby forming the first rough surface in the exposed first roughening region. In the present disclosure, the manufacturing method may further include a step of removing the third protective material after the first roughening step and before the second roughening step. In the second roughening step, a fourth protective material may be disposed to protect the non-roughening region while exposing the second roughening region with respect to the base 10 to form the second rough surface on the exposed second roughening region. The second roughening step may be performed such that the surface of the second rough surface formed in the second roughening step has a surface roughness smaller than that of the first rough surface in the first roughening region. In the present disclosure, the manufacturing method may include a step of removing the fourth protective material after the second roughening step and before the coating film forming step. For example, a fourth masking tape may be used as the fourth protective material. In this case, in the present disclosure, the manufacturing method may further include a step of attaching a fourth masking tape to the non-roughening region while exposing the first roughening region and the second roughening region before the second roughening step. As the fourth protective material, a material having lower thermal resistance than the above-described third protective material may be used. For example, a material that is not dissolved or pyrolyzed at room temperature may be used as the fourth protective material. Specifically, a resin may be used as the fourth protective material.

In the second roughening step, the non-roughening region may or may not be covered with a protective material. The first roughening region and the non-roughening region may be protected, and only the second roughening region may be processed by at least one selected from the group consisting of chemical etching method and blasting method. Alternatively, a fourth protective material may be disposed so as to expose the first roughening region, and the rough surface of the exposed first roughening region and the second roughening region may be processed by at least one selected from the group consisting of a chemical etching method and a blasting method. Thus, an excess thermal spraying material or the like remaining on the rough surface of the first roughening region can be removed, and a rough surface can be also formed on the second roughening region.

The method of manufacturing the artificial joint stem 100 may initially include a step of preparing a base 10 in which the first roughening region, the second roughening region, and the non-roughening region are located in this order.

### 3. Use of Artificial Joint Stem

Although the artificial joint stem 100 illustrated in FIG. 1 has a shape mainly assuming a stem for an artificial hip j oint, artificial joints to which the artificial joint stem according to the present disclosure is applied, are not limited to artificial hip joints. Examples of the artificial joint include an artificial hip joint, an artificial knee joint, an artificial ankle joint, an artificial shoulder j oint, an artificial elbow joint and an artificial finger joint.

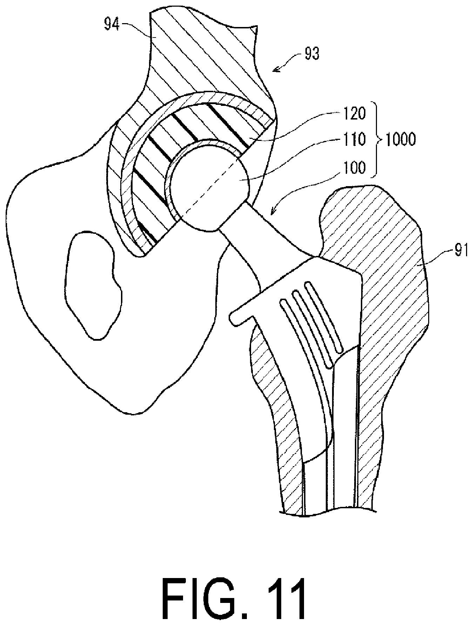

Referring now to FIG. 11, an example in which the artificial joint stem 100 is used as a part of an artificial hip joint 1000 will be described below. The artificial hip joint 1000 may include a bone head 110 and an acetabular cup 120 in addition to the artificial joint stem 100. The bone head 110 and the acetabular cup 120 may be formed of the same or different materials as the base 10 of the artificial joint stem 100. The artificial joint stem 100 is embedded in a femur 91. The bone head 110 is disposed at the exposed portion 50 of the artificial joint stem 100. The acetabular cup 120 is fixed to an acetabular 94 of a hip bone 93. The acetabular cup functions as a hip joint by fitting and sliding the bone head 110 into the depression of the acetabular cup 120.

In the present disclosure, the invention has been described above based on the various drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, note that a person skilled in the art can easily make various variations or modifications based on the present disclosure. Note that these variations or modifications are included within the scope of the present disclosure.

### REFERENCE SIGNS

1 First groove
2 Second groove
10 Base
20 Coating film
100 Artificial joint stem
1000 Artificial hip joint

## Claims

1. An artificial joint stem comprising:
a base comprising one or more grooves disposed on a surface of the base; and
a coating film containing a calcium phosphate-based material and an antimicrobial material disposed on a part of the surface of the base,
wherein
when among the one or more grooves, the groove located in a region where the coating film is disposed is defined as a first groove, and the groove located in a region where the surface of the base is exposed from the coating film is defined as a second groove, a total length of the first groove is smaller than a total length of the second groove.

2. The artificial joint stem according to claim 1, wherein
the first groove is connected to the second groove.

3. The artificial joint stem according to claim 1 or 2, wherein
the depth of the first groove is smaller than the depth of the second groove.

4. The artificial joint stem according to any one of claims 1 to 3, wherein
the width of the first groove is smaller than the width of the second groove.

5. The artificial joint stem according to any one of claims 1 to 4, wherein
the one or more grooves comprise a plurality of first grooves.

6. The artificial joint stem according to any one of claims 1 to 5, wherein
the first groove has a component along a width direction of the base.

7. The artificial joint stem according to any one of claims 1 to 6, wherein
in the first groove, a component along the width direction of the base is smaller than a component along a direction orthogonal to the width direction.

8. The artificial joint stem according to any one of claims 1 to 7, wherein
the first groove comprises a plurality of linear portions.

9. The artificial joint stem according to claim 8, wherein
the plurality of linear portions intersect each other at a right angle.

10. The artificial joint stem according to any one of claims 1 to 9, wherein
in the second groove, a component along the width direction of the base is smaller than a component along a direction orthogonal to the width direction.

11. The artificial joint stem according to claim 1, wherein
the one or more grooves comprise only the second groove.

12. The artificial joint stem according to any one of claims 1 to 11, wherein
at least one of the one or more grooves each comprises one end portion and the other end portion in a width direction of the respective groove, and a depth at the one end portion is smaller than a depth at the other end portion.

13. The artificial joint stem according to any one of claims 1 to 12, wherein
among the surface of the base, an area of a region where the coating film is disposed is greater than an area of a region where the surface of the base is exposed from the coating film.

14. The artificial joint stem according to any one of claims 1 to 13, wherein
within the surface of the base, a length along an orthogonal direction orthogonal to the width direction of the base in the region where the coating film is disposed, is smaller than a length along the orthogonal direction in the region where the surface of the base is exposed from the coating film.

15. The artificial joint stem according to any one of claims 1 to 10, wherein
the base comprises a rough surface located on the surface, and only the first groove is located on the rough surface.

16. An artificial joint stem comprising:
a base comprising one or more recesses disposed on a surface of the base; and
a coating film containing a calcium phosphate-based material and an antimicrobial material disposed on a part of the surface of the base,
wherein
when the recess located in a region where the coating film is disposed is defined as a first recess, and the recess located in a region where the surface of the base is exposed from the coating film is defined as a second recess, a total opening area of the first recess is smaller than a total opening area of the second recess.
